# EUROPEAN PATENT APPLICATION

(11) **EP 4 760 349 A1**
(43) Date of publication of application: **17.06.2026**
(21) Application number: 24851861.5
(22) Date of filing: 06.08.2024
(51) Int. Cl.: G01S 13/34, A61B 5/02, A61B 5/11, A61B 5/0245, G01S 7/34, G01S 13/58

(54) **ELECTRONIC DEVICE, METHOD FOR CONTROLLING ELECTRONIC DEVICE, AND PROGRAM**

(30) Priority: 09.08.2023 JP 2023130366
(71) Applicant: Kyocera Corporation, Kyoto-shi, Kyoto 612-8501 (JP)
(72) Inventor: KANDA, Toi, Kyoto-shi, Kyoto 612-8501 (JP); ASAI, Morisuke, Kyoto-shi, Kyoto 612-8501 (JP); KURODA, Jun, Kyoto-shi, Kyoto 612-8501 (JP)
(74) Representative: Viering, Jentschura & Partner mbB Patent- und Rechtsanwälte
(86) International application number: PCT/JP2024/028121
(87) International publication number: WO 2025/033438

(57) **Abstract**

An electronic device is provided. The electronic device includes a signal processing unit. The signal processing unit is configured to detect vibrations in a subject of monitoring. The detection is based on a transmission signal transmitted as a transmission wave and a received signal received as a reflected wave resulting from the transmission wave being reflected by the subject of monitoring. The signal processing unit is configured to generate first information related to a heart sound of the subject of monitoring on the basis of vibrations in a first part of the subject of monitoring. If the first information does not satisfy a prescribed criterion, the signal processing unit is configured to generate second information related to a pulse wave of the subject of monitoring on the basis of vibrations in a second part, which is different from the first part, of the subject of monitoring.

## Description

### CROSS-REFERENCE TO RELATED APPLICATIONS

This application claims priority based on Japanese Patent Application No. 2023-130366 filed August 9, 2023, the entire disclosure of which is hereby incorporated by reference.

### TECHNICAL FIELD

The present disclosure relates to an electronic device, a method of controlling an electronic device, and a program.

### BACKGROUND OF INVENTION

Various technologies have been proposed to determine possible risks to a person to be monitored using a sensor that acquires biological information about the human body, such as a biosensor. For example, Patent Literature 1, Patent Literature 2, and Patent Literature 3 each propose a technology for detecting an abnormality such as a person drowning in bathtub water (hot water) while taking a bath.

In fields such as the automotive and related industries, for example, technologies for measuring values such as the distance between a vehicle and a given object are gaining importance. In particular, recent years have seen various research into radio detection and ranging (RADAR) technology, which measures values such as the distance to an obstacle or other object by transmitting radio waves, such as millimeter waves, for example, and receiving reflected waves back from the object. In this way, the importance of technologies for measuring distances and the like is expected to increase further with the development of technologies for assisting drivers with driving and technologies related to self-driving, in which driving is partially or fully automated. Research is underway to apply such radar technology to the detection of vital signs such as the pulse of a person to be monitored.

### CITATION LIST

### PATENT LITERATURE

Patent Literature 1: Japanese Unexamined Patent Application Publication No. 2019-135595
Patent Literature 2: Japanese Unexamined Patent Application Publication No. 2019-135596
Patent Literature 3: Japanese Unexamined Patent Application Publication No. 2020-22678

### SUMMARY

In an embodiment, an electronic device is provided. The electronic device includes a signal processing unit. The signal processing unit is configured to detect vibrations in a subject of monitoring. The detection is based on a transmission signal transmitted as a transmission wave and a received signal received as a reflected wave resulting from the transmission wave being reflected by the subject of monitoring. The signal processing unit is configured to generate first information related to a heart sound of the subject of monitoring on the basis of vibrations in a first part of the subject of monitoring. If the first information does not satisfy a prescribed criterion, the signal processing unit is configured to generate second information related to a pulse wave of the subject of monitoring on the basis of vibrations in a second part, which is different from the first part, of the subject of monitoring.

In an embodiment, a method of controlling an electronic device is provided. The electronic device is provided with a signal processing unit configured to detect vibrations in a subject of monitoring. The detection is based on a transmission signal transmitted as a transmission wave and a received signal received as a reflected wave resulting from the transmission wave being reflected by the subject of monitoring. The method includes generating first information related to a heart sound of the subject of monitoring on the basis of vibrations in a first part of the subject of monitoring. The method includes generating, if the first information does not satisfy a prescribed criterion, second information related to a pulse wave of the subject of monitoring on the basis of vibrations in a second part, which is different from the first part, of the subject of monitoring.

In an embodiment, a program is provided. The program causes an electronic device to execute processing. The electronic device is provided with a signal processing unit configured to detect vibrations in a subject of monitoring. The detection is based on a transmission signal transmitted as a transmission wave and a received signal received as a reflected wave resulting from the transmission wave being reflected by the subject of monitoring. The processing includes generating first information related to a heart sound of the subject of monitoring on the basis of vibrations in a first part of the subject of monitoring. The processing includes generating, if the first information does not satisfy a prescribed criterion, second information related to a pulse wave of the subject of monitoring on the basis of vibrations in a second part, which is different from the first part, of the subject of monitoring.

### BRIEF DESCRIPTION OF THE DRAWINGS

FIG. 1 is a diagram for describing how an electronic device according to an embodiment is used.
FIG. 2 is a function block diagram schematically illustrating a configuration of an electronic device according to an embodiment.
FIG. 3 is a diagram for describing the composition of a signal processed by an electronic device according to an embodiment.
FIG. 4 is a diagram for describing the processing of a signal by an electronic device according to an embodiment.
FIG. 5 is a diagram for describing the processing of a signal by an electronic device according to an embodiment.
FIG. 6 is a diagram for describing the processing of a signal by an electronic device according to an embodiment.
FIG. 7 schematically illustrates an example of the antenna arrangement and the operating principle of an antenna array of an electronic device according to an embodiment.
FIG. 8 illustrates an example of the antenna arrangement in an antenna array of an electronic device according to an embodiment.
FIG. 9 is a diagram for describing an example of a usage environment of an electronic device according to an embodiment.
FIG. 10 is a diagram for describing an example of a usage environment of an electronic device according to an embodiment.
FIG. 11 is a flowchart for describing operations by an electronic device according to an embodiment.
FIGs. 12A to 12C are graphs illustrating an example of operations by an electronic device according to an embodiment.
FIG. 13 is a graph illustrating an enlarged view of a portion of the graph illustrated in FIG. 12A.
FIGs. 14A to 14C is a graph illustrating an example of operations by an electronic device according to an embodiment.
FIG. 15 is a graph illustrating an enlarged view of a portion of the graph illustrated in FIG. 14A.

### DESCRIPTION OF EMBODIMENTS

The ability to increase the likelihood of detecting biological information such as the heartbeat of a subject of monitoring by transmitting and receiving radio waves, such as millimeter waves for example, could be useful in a wide variety of fields. An objective of the present disclosure is to provide an electronic device, a method of controlling an electronic device, and a program that may be implemented to increase the likelihood of detecting biological information such as the heartbeat of a subject of monitoring by transmitting and receiving radio waves. According to an embodiment, it is possible to provide an electronic device, a method of controlling an electronic device, and a program that may be implemented to increase the likelihood of detecting biological information such as the heartbeat of a subject of monitoring by transmitting and receiving radio waves.

In the present disclosure, an "electronic device" may be a device driven by electric power. A "user" may be an entity (typically a human being) or an animal that uses an electronic device according to an embodiment and/or a system including the electronic device. The user may include an entity that monitors a human being or other subject by using an electronic device according to an embodiment, or a system including the electronic device. The "subject" may be an entity to be monitored (hereinafter also referred to as the "subject of monitoring") using an electronic device according to an embodiment. The subject of monitoring may be a human being or an animal, for example. The user may also include the subject of monitoring.

In the present disclosure, "heartbeat" may refer to the pulsation of the heart. "Pulsation" may refer to a rhythmic contraction movement of the heart or an artery. The heart may be a human heart or an animal heart. The "heartbeat" and the "heart sound" may be the "heartbeat" and the "heart sound" of a human or an animal.

In the present disclosure, "heart rate" refers to the number of times the heart pulsates in a prescribed period of time. For example, the heart rate may refer to the number of pulsations per minute. Pulsations occur in the arteries when the heart pumps blood. Accordingly, the number of pulsations by the arteries may also be referred to as the "pulse rate" or simply the "pulse".

In the present disclosure, "heart sound" may refer to the sound of a beating heart. That is, the heart sound may refer to the sound that occurs when the heart contracts and dilates. The "heart sound" may include a low, long first sound based on ventricular muscle tension, mitral valve closure, initiation of blood ejection into the arteries, and/or acceleration of blood flow, followed by a high, short second sound derived from aortic valve closure and/or pulmonary valve closure.

In the present disclosure, the heart sound is not necessarily limited to physical sounds based on air vibrations, and may also mean the vibrations themselves caused by the beating (pulsation) of the heart. For example, in the present disclosure, the heartbeat may be assumed to imply a vibrating source, and the heart sound may be assumed to imply the vibrations themselves caused by the vibrating source. In the present disclosure, the heartbeat may also be assumed to imply the heart sound, depending on the situation.

In the present disclosure, a "pulse wave" may be defined as a waveform expression of a change in the volume of a blood vessel caused by the heart pumping blood.

An electronic device according to an embodiment can detect the heartbeat of a human being or other subject of monitoring present in the surroundings of the electronic device. Accordingly, anticipated situations in which an electronic device according to an embodiment is used may be, for example, specific facilities or the like used by people engaged in social activities, such as residences, companies, hospitals, nursing homes, schools, sports gyms, and nursing care facilities. For example, it is extremely important for a company to grasp and/or manage the health status of its employees and the like. Similarly, it is extremely important for a hospital to grasp and/or manage the health status of its patients, medical personnel, and the like, and for a nursing home to grasp and/or manage the health status of its residents, staff, and the like. The situations in which an electronic device according to an embodiment is used are not limited to facilities such as companies, hospitals, and nursing homes such as those described above, and may be any given facility where it is desirable to grasp and/or manage the health status of a subject of monitoring. The any given facility may also include a non-commercial facility, such as the home of a user. The situations in which an electronic device according to an embodiment is used are not limited to indoors and may also be outdoors. For example, the situations in which an electronic device according to an embodiment is used may also be inside means of transportation such as trains, buses, airplanes, and the like, as well as stations, boarding areas, and the like. The situations in which an electronic device according to an embodiment is used may also be a means of transportation such as an automobile, aircraft, or ship, a hotel, the home of a user, or the living room, bath, toilet, bedroom, or the like in a home. The situations in which an electronic device according to an embodiment is used may also be when measuring the heartbeats of animals such as cows, pigs, or other livestock at zoos, ranches, farms, and the like.

In a nursing facility, for example, an electronic device according to an embodiment may be used for the purpose of detecting or monitoring the heartbeat of a subject of monitoring such as a person in need of nursing or care. If an abnormality is found in the heartbeat of a subject of monitoring such as a person in need of nursing or care, for example, an electronic device according to an embodiment may also issue a predetermined warning to the person in question and/or another person. Consequently, according to an electronic device according to an embodiment, the person in question and/or a staff member of a nursing care facility or the like may recognize that an abnormality is found in the pulse of a subject of monitoring such as a person in need of nursing or care, for example. On the other hand, if an abnormality is not found (for example, the heartbeat is found to be normal) in the heartbeat of a subject of monitoring such as a person in need of nursing or care, for example, an electronic device according to an embodiment may also issue a notification to that effect to the person in question and/or another person. Consequently, according to an electronic device according to an embodiment, the person in question and/or a staff member of a nursing care facility or the like may recognize that the pulse is normal for a subject of monitoring such as a person in need of nursing or care, for example. An electronic device according to an embodiment may also use the heartbeat of a subject of monitoring for estimation of the heartbeat interval (the RR interval (RRI) in an electrocardiogram), detection of dozing off and estimation of predictive signs thereof, estimation of level of alertness, estimation of level of fatigue, or identification of the subject of monitoring.

An electronic device according to an embodiment may also detect the pulse of an animal other than a human being as the subject of monitoring. As an example, the electronic device according to an embodiment described hereinafter is described as detecting the pulse of a human being using a sensor based on a technology like millimeter-wave radar.

An electronic device according to an embodiment may be installed in any stationary object, and may also be installed in any moving object. An electronic device according to an embodiment can use a transmitting antenna to transmit a transmission wave to the surroundings of the electronic device. An electronic device according to an embodiment can use a receiving antenna to receive a reflected wave resulting from the transmission wave being reflected. Each of the transmitting antenna and the receiving antenna may also be an antenna array (array antenna) configured to include a plurality of antenna elements. At least one of the transmitting antenna or the receiving antenna may be provided in the electronic device, and may also be provided in a radar sensor, for example.

In the following, an electronic device according to an embodiment is described as stationary. An electronic device according to an embodiment may also be installed in a moving body such as an automobile, for example. By being installed in a moving body such as an automobile, for example, an electronic device according to an embodiment may detect the heartbeat and the like of an occupant on board the moving body. On the other hand, the subject of monitoring (human being) whose pulse is to be detected by an electronic device according to an embodiment may be stationary, moving, or moving their body while remaining stationary. Like an ordinary radar sensor, an electronic device according to an embodiment can measure the distance and the like between the electronic device and an object in the surroundings of the electronic device in situations in which the object may move. An electronic device according to an embodiment can measure the distance and the like between the electronic device and an object even if the electronic device and the object are both stationary.

The following describes an electronic device according to an embodiment in detail, with reference to the drawings. First, an example of the detection of an object by an electronic device according to an embodiment will be described.

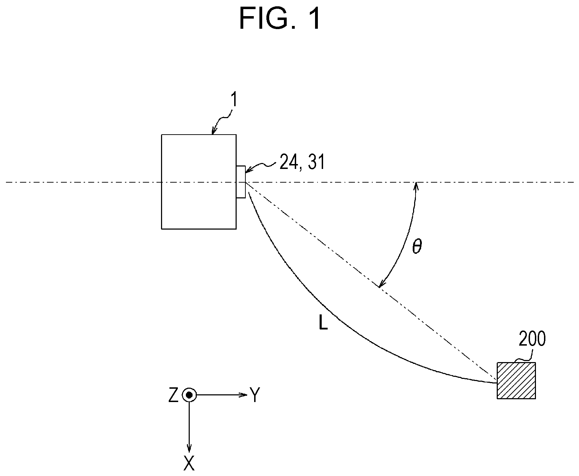

FIG. 1 is a diagram for describing an example of how an electronic device according to an embodiment is used. FIG. 1 illustrates an example of an electronic device provided with the functions of a sensor provided with a transmitting antenna and a receiving antenna according to an embodiment.

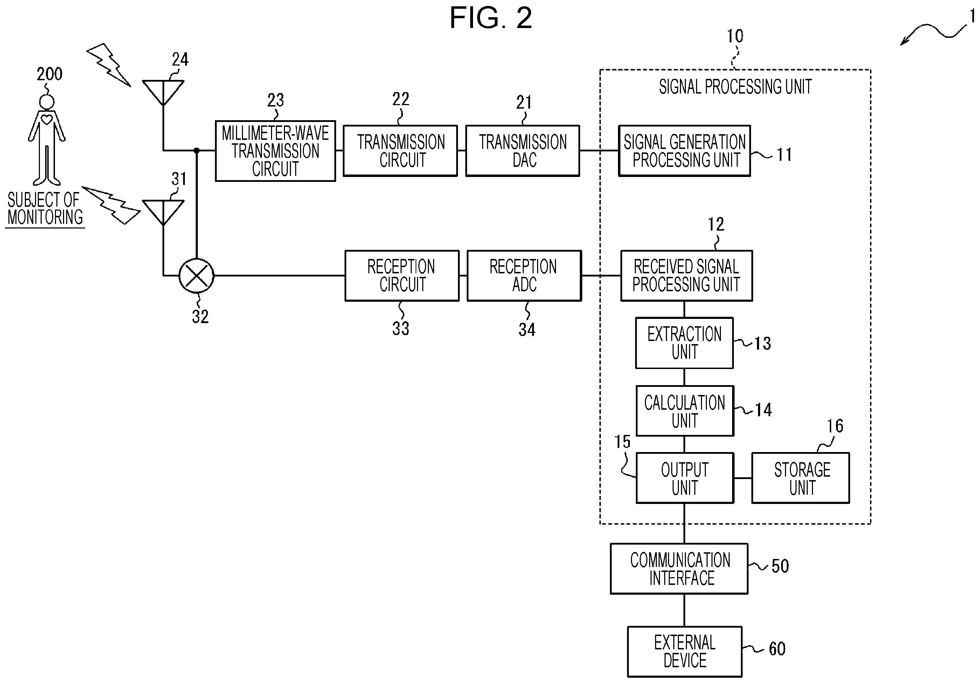

As illustrated in FIG. 1, in an embodiment, an electronic device 1 may be provided with a transmission unit and a reception unit. As described later, the transmission unit may be provided with a transmitting antenna array 24. The reception unit may be provided with a receiving antenna array 31. Specific configurations of the electronic device 1, the transmission unit, and the reception unit will be described later. For simplicity, FIG. 1 illustrates a situation in which the electronic device 1 is provided with the transmitting antenna array 24 and the receiving antenna array 31. The electronic device 1 may also include at least one other functional unit, as appropriate, such as at least a portion of a signal processing unit 10 (FIG. 2) described later. The electronic device 1 may be provided with at least one other functional unit outside the electronic device 1, such as at least a portion of a signal processing unit 10 (FIG. 2) included in the electronic device 1. In FIG. 1, the electronic device 1 may be moving, but may also be stationary without moving. FIG. 1 schematically illustrates functional units such as the electronic device 1.

The example illustrated in FIG. 1 illustrates in a simplified manner the transmission unit provided with the transmitting antenna array 24 and the reception unit provided with the receiving antenna array 31 in the electronic device 1. The electronic device 1 may also be provided with a plurality of transmission units and/or a plurality of reception units, for example. The transmission unit may be provided with a transmitting antenna array 24 formed from a plurality of transmitting antennas. The reception unit may be provided with a receiving antenna array 31 formed from a plurality of receiving antennas. The position where the transmission unit and/or reception unit are installed in the electronic device 1 is not limited to the position illustrated in FIG. 1, and may be another position, as appropriate. The number of transmission units and/or reception units may be any number equal to or greater than 1, according to various conditions (or requirements) such as the range and/or precision of heartbeat detection by the electronic device 1.

As described later, the electronic device 1 transmits an electromagnetic wave as a transmission wave from the transmitting antenna array 24. For example, if a given object (for example, the subject of monitoring 200 illustrated in FIG. 1) is present in the surroundings of the electronic device 1, at least a portion of the transmission wave transmitted from the electronic device 1 is reflected by the object to become a reflected wave. Such a reflected wave is then received by the receiving antenna array 31 of the electronic device 1, for example, whereby the electronic device 1 can detect the subject of monitoring as a target.

Typically, the electronic device 1 provided with the transmitting antenna array 24 may be a radio detection and ranging (RADAR) sensor that transmits and receives radio waves. However, the electronic device 1 is not limited to a radar sensor. In an embodiment, the electronic device 1 may also be, for example, a sensor based on light detection and ranging (LIDAR) technology, also known as laser imaging detection and ranging, which involves light waves. Sensors such as these can be configured to include a patch antenna or the like. Since technologies such as RADAR and LIDAR are already known, a detailed description of these technologies may be simplified or omitted, as appropriate. In an embodiment, the electronic device 1 may also be a sensor based on a technology that detects objects by transmitting and receiving sonic or ultrasonic waves, for example.

The electronic device 1 illustrated in FIG. 1 receives from the receiving antenna array 31 a reflected wave of a transmission wave transmitted from the transmitting antenna array 24. With this arrangement, the electronic device 1 can detect a given subject of monitoring 200 present within a given distance from the electronic device 1 as a target. For example, as illustrated in FIG. 1, the electronic device 1 can measure the distance L between the electronic device 1 and a given subject of monitoring 200. The electronic device 1 can also measure the relative velocity between the electronic device 1 and a given subject of monitoring 200. The electronic device 1 can also measure the direction (angle of arrival θ) from which the reflected wave from a given subject of monitoring 200 arrives at the electronic device 1.

In FIG. 1, the XY plane may be defined as the plane substantially parallel to the ground, for example. In this case, the positive direction of the Z axis illustrated in FIG. 1 may indicate the vertically upward direction. In FIG. 1, the electronic device 1 may be installed on a plane parallel to the XY plane. In FIG. 1, the subject of monitoring 200 may be standing on the ground substantially parallel to the XY plane, for example.

The subject of monitoring 200 may be a human being or the like present in the surroundings of the electronic device 1, for example. The subject of monitoring 200 may also be a living thing other than a human being, such as an animal, present in the surroundings of the electronic device 1, for example. As described above, the subject of monitoring 200 may be moving, and may also be stopped or stationary. In the present disclosure, the object to be detected by the electronic device 1 includes inanimate things such as any object, as well as living things such as people, dogs, cats, horses, and other animals. The object to be detected by the electronic device 1 according to the present disclosure may also include a target, including a person, a thing, an animal, or the like, that is detected by radar technology. In the present disclosure, a target may include a person, a thing, an animal, or the like. The following description assumes that the object (target) such as the subject of monitoring 200 present in the surroundings of the electronic device 1 is a human being. However, as described above, in an embodiment, the object (target) that the electronic device 1 detects may also be an animal. In the present disclosure, the target may also be the subject of monitoring 200 above.

In FIG. 1, the ratio of the size of the electronic device 1 to the size of the subject of monitoring 200 does not necessarily indicate the actual ratio. In FIG. 1, the transmitting antenna array 24 of the transmission unit and the receiving antenna array 31 of the reception unit are illustrated as being installed on the outside of the electronic device 1. However, in an embodiment, the transmitting antenna array 24 of the transmission unit and/or the receiving antenna array 31 of the reception unit may be installed at various positions in the electronic device 1. For example, in an embodiment, the transmitting antenna array 24 of the transmission unit and/or the receiving antenna array 31 of the reception unit may be installed inside the electronic device 1, and may not appear on the exterior of the electronic device 1.

The following describes a typical example in which the transmitting antenna of the electronic device 1 transmits radio waves in a frequency band such as millimeter waves (30 GHz or higher) or quasi-millimeter waves (for example, around 20-30 GHz). On the other hand, the transmitting antenna of the electronic device 1 may also transmit radio waves with a frequency bandwidth of 4 GHz, such as from 77 GHz to 81 GHz, for example. The transmitting antenna of the electronic device 1 may also transmit radio waves of high frequency (for example, 30 GHz to 300 GHz) in or above the millimeter-wave band.

FIG. 2 is a function block diagram schematically illustrating an example configuration of the electronic device 1 according to an embodiment. The following describes an example of the configuration of the electronic device 1 according to an embodiment.

Frequency-modulated continuous-wave radar (hereinafter referred to as FMCW radar) is often used to measure distances and the like using millimeter-wave radar. In FMCW radar, a transmission signal is generated by sweeping the frequencies of the radio waves to be transmitted. Accordingly, in a millimeter-wave FMCW radar that uses radio waves in the 79 GHz frequency band, for example, the frequencies of the radio waves to be used have a frequency bandwidth of 4 GHz, such as from 77 GHz to 81 GHz, for example. Radar in the 79 GHz frequency band is characterized by having a wider usable frequency bandwidth than other millimeter/quasi-millimeter wave radars, such as radars in the 24 GHz, 60 GHz, and 76 GHz frequency bands, for example. The following describes such an embodiment as an example.

The radar scheme of the FMCW radar used in the present disclosure may include a fast-chirp modulation (FCM) scheme that transmits chirp signals on a shorter period than usual. The signal that the electronic device 1 generates is not limited to a signal of the FMCW scheme. The signal that the electronic device 1 generates may also be a signal of any of various schemes other than the FMCW scheme. A transmission signal sequence stored in any storage unit may be different depending on these various schemes. For example, in the case of a radar signal of the FMCW scheme described above, a signal of increasing frequency and a signal of decreasing frequency at each time sample may be used. Known technologies can be applied, as appropriate, for the various schemes described above, and thus a more detailed description is omitted.

As illustrated in FIG. 2, in an embodiment, the electronic device 1 is provided with a signal processing unit 10. The signal processing unit 10 may be provided with a signal generation processing unit 11, a received signal processing unit 12, an extraction unit 13, a calculation unit 14, an output unit 15, and a storage unit 16. In an embodiment, the signal processing unit 10 may also not include a portion of the functional units illustrated in FIG. 2, and may also include functional units other than the functional units illustrated in FIG. 2.

In the signal processing unit 10, the extraction unit 13 may execute processing to extract a micro-Doppler component, for example. The extraction unit 13 may also execute processing to extract an envelope of the heart sound of the subject of monitoring 200. The calculation unit 14 may execute processing to calculate a heartbeat interval (RRI) of the subject of monitoring 200, for example. The calculation unit 14 may also execute processing to calculate the heartbeat of the subject of monitoring 200, for example. The calculation unit 14 may further execute processing to calculate a heart rate variability (HRV) of the subject of monitoring 200, for example. The output unit 15 outputs a result of calculation by the calculation unit 14 from the signal processing unit 10, as appropriate. In this case, the output unit 15 may output a result of calculation by the calculation unit 14 if the result meets a condition stored in the storage unit 16. The storage unit 16 may store various information. In particular, the storage unit 16 may store information that serves as at least one criterion for determining whether the output unit 15 is to output certain information. The signal generation processing unit 11, received signal processing unit 12, extraction unit 13, calculation unit 14, output unit 15, and storage unit 16 will be further described later, as appropriate. The storage unit 16 may include a storage device of any kind, such as random access memory (RAM) and read-only memory (ROM), for example. The storage unit 16 stores various programs for causing the signal processing unit 10 to exhibit functionality and various information to be used by the signal processing unit 10. An embodiment in the form of a program is not limited to an application program such as object code compiled by a compiler or program code to be executed by an interpreter. An embodiment in the form of a program may also be in a form such as a program module incorporated into an operating system. The program may or may not be configured so that all processing is performed solely in a CPU on a control board. The program to be used in the present disclosure may also be configured to be implemented, in part or in full, by another processing unit mounted on an expansion board or expansion unit added to the board as needed.

In the present disclosure, the heart sound may refer to, for example, a vibration waveform of a chest area or the like observed directly by radar, or to chest vibrations. The heartbeat refers to the pulsations themselves of the heart. A heartbeat interval, heart rate, or the like may be calculated from the movement of the heartbeat. The heartbeat interval may refer to the time interval between a pulsation of the heart and the next pulsation of the heart.

In an embodiment, the electronic device 1 is provided with a transmission DAC 21, a transmission circuit 22, a millimeter-wave transmission circuit 23, and the transmitting antenna array 24 as the transmission unit. In an embodiment, the electronic device 1 is provided with the receiving antenna array 31, a mixer 32, a reception circuit 33, and a reception ADC 34 as the reception unit. In an embodiment, the electronic device 1 need not include at least one of the functional units illustrated in FIG. 2, and may also include a functional unit other than the functional units illustrated in FIG. 2. The electronic device 1 illustrated in FIG. 2 may be formed using a circuit configured in basically the same and/or similar way as a common radar using electromagnetic waves in the millimeter-wave band or the like. On the other hand, in the electronic device 1 according to an embodiment, the signal processing by the signal processing unit 10 may include processing different from the processing performed by a common radar of the related art.

In an embodiment, the signal processing unit 10 provided in the electronic device 1 can control operations by the electronic device 1 as a whole, including control of each of the functional units that make up the electronic device 1. In particular, the signal processing unit 10 performs various processing with respect to signals handled by the electronic device 1. To provide control and processing power for executing various functions, the signal processing unit 10 may include at least one processor, such as a central processing unit (CPU) or a digital signal processor (DSP). The signal processing unit 10 may be realized entirely with a single processor, with several processors, or with respectively discrete processors. The processor may be realized as a single integrated circuit. An integrated circuit is also referred to as an IC. The processor may be realized as a plurality of communicatively connected integrated circuits and discrete circuits. The processor may be realized on the basis of any of various other known technologies. In an embodiment, the signal processing unit 10 may be configured as a CPU (hardware) and a program (software) executed by the CPU, for example. The signal processing unit 10 may include a storage unit (memory) required for operations by the signal processing unit 10.

The signal generation processing unit 11 of the signal processing unit 10 generates a signal to be transmitted from the electronic device 1. In the electronic device 1 according to an embodiment, the signal generation processing unit 11 may generate a transmission signal such as a chirp signal (transmission chirp signal). In particular, the signal generation processing unit 11 may generate signals (linear chirp signals) whose frequency varies periodically and linearly. For example, the signal generation processing unit 11 may generate chirp signals whose frequency increases periodically and linearly from 77 GHz to 81 GHz over time. As another example, the signal generation processing unit 11 may generate a signal whose frequency periodically and linearly increases (up-chirp) from 77 GHz to 81 GHz and then decreases (down-chirp) over time. The signal that the signal generation processing unit 11 generates may also be preset in the signal processing unit 10, for example. The signal that the signal generation processing unit 11 generates may also be stored in advance in any storage unit or the like in the signal processing unit 10, for example. Since chirp signals used in technical fields such as radar are already known, a more detailed description is simplified or omitted, as appropriate. The signal generated by the signal generation processing unit 11 is supplied to the transmission DAC 21. For this reason, the signal generation processing unit 11 may be connected to the transmission DAC 21.

The transmission DAC (digital-to-analog converter) 21 functions to convert a digital signal supplied from the signal generation processing unit 11 to an analog signal. The transmission DAC 21 may include a common digital-to-analog converter. The signal converted to analog by the transmission DAC 21 is supplied to the transmission circuit 22. For this reason, the transmission DAC 21 may be connected to the transmission circuit 22.

The transmission circuit 22 functions to convert the signal converted to analog by the transmission DAC 21 to an intermediate frequency (IF) band. The transmission circuit 22 may include a common IF-band transmission circuit. The signal processed by the transmission circuit 22 is supplied to the millimeter-wave transmission circuit 23. For this reason, the transmission circuit 22 may be connected to the millimeter-wave transmission circuit 23.

The millimeter-wave transmission circuit 23 functions to transmit the signal processed by the transmission circuit 22 as a millimeter wave (RF wave). The millimeter-wave transmission circuit 23 may include a common millimeter-wave transmission circuit. The signal processed by the millimeter-wave transmission circuit 23 is supplied to the transmitting antenna array 24. For this reason, the millimeter-wave transmission circuit 23 may be connected to the transmitting antenna array 24. The signal processed by the millimeter-wave transmission circuit 23 is also supplied to the mixer 32. For this reason, the millimeter-wave transmission circuit 23 may also be connected to the mixer 32.

The transmitting antenna array 24 is a plurality of transmitting antennas arranged into an array. In FIG. 2, the configuration of the transmitting antenna array 24 is illustrated in a simplified manner. The transmitting antenna array 24 transmits a signal processed by the millimeter-wave transmission circuit 23 to the outside of the electronic device 1. The transmitting antenna array 24 may include a transmitting antenna array used in a common millimeter-wave radar.

In this way, in an embodiment, the electronic device 1 is provided with a transmitting antenna (transmitting antenna array 24) and can transmit a transmission signal (transmission chirp signal, for example) as a transmission wave from the transmitting antenna array 24.

As an example, suppose the case in which an object such as the subject of monitoring 200 is present in the surroundings of the electronic device 1, as illustrated in FIG. 2. In this case, at least a portion of the transmission wave transmitted from the transmitting antenna array 24 is reflected by an object such as the subject of monitoring 200. The at least a portion of the transmission wave transmitted from the transmitting antenna array 24 that is reflected by an object such as the subject of monitoring 200 may be reflected toward the receiving antenna array 31.

The receiving antenna array 31 receives a reflected wave. The reflected wave may refer to at least a portion of a transmission wave transmitted from the transmitting antenna array 24 that is reflected by an object such as the subject of monitoring 200.

The receiving antenna array 31 is a plurality of receiving antennas arranged into an array. In FIG. 2, the configuration of the receiving antenna array 31 is illustrated in a simplified manner. The receiving antenna array 31 receives a reflected wave resulting from a transmission wave transmitted from the transmitting antenna array 24 being reflected. The receiving antenna array 31 may include a receiving antenna array used in a common millimeter-wave radar. The receiving antenna array 31 supplies a received signal received as a reflected wave to the mixer 32. For this reason, the receiving antenna array 31 may be connected to the mixer 32.

The mixer 32 converts a signal (transmission signal) processed by the millimeter-wave transmission circuit 23 and a received signal received by the receiving antenna array 31 to an intermediate frequency (IF) band. The mixer 32 may include a mixer used in a common millimeter-wave radar. The mixer 32 supplies a signal generated as a synthesized result to the reception circuit 33. For this reason, the mixer 32 may be connected to the reception circuit 33.

The reception circuit 33 functions to perform analog processing on a signal converted to an IF band by the mixer 32. The reception circuit 33 may include a common reception circuit that performs conversion to an IF band. The signal processed by the reception circuit 33 is supplied to the reception ADC 34. For this reason, the reception circuit 33 may be connected to the reception ADC 34.

The reception ADC (analog-to-digital converter) 34 functions to convert an analog signal supplied from the reception circuit 33 to a digital signal. The reception ADC 34 may include a common analog-to-digital converter. The signal converted to digital by the reception ADC 34 is supplied to the received signal processing unit 12 of the signal processing unit 10. For this reason, the reception ADC 34 may be connected to the signal processing unit 10.

The received signal processing unit 12 of the signal processing unit 10 functions to perform various processing on a digital signal supplied from the reception ADC 34. For example, the received signal processing unit 12 calculates the distance from the electronic device 1 to an object such as the subject of monitoring 200 on the basis of a digital signal supplied from the reception ADC 34 (distance measurement). The received signal processing unit 12 also calculates the relative velocity of an object such as the subject of monitoring 200 relative to the electronic device 1 on the basis of a digital signal supplied from the reception ADC 34 (velocity measurement). The received signal processing unit 12 further calculates the bearing angle of an object such as the subject of monitoring 200 as seen from the electronic device 1 on the basis of a digital signal supplied from the reception ADC 34 (angle measurement). Specifically, I/Q converted data may be inputted into the received signal processing unit 12. By accepting the input of such data, the received signal processing unit 12 performs a fast Fourier transform (2D-FFT) in each of the distance (range) and velocity directions. The received signal processing unit 12 may then perform false alarm suppression and fixed probability conversion by removing noise points through processing such as constant false alarm rate (CFAR). The received signal processing unit 12 can perform angle-of-arrival estimation for points that satisfy the CFAR criterion to obtain the position of an object such as the subject of monitoring 200. The information generated as a result of the distance measurement, velocity measurement, and angle measurement by the received signal processing unit 12 may be supplied to the extraction unit 13.

The extraction unit 13 extracts information related to heartbeat from information generated by the received signal processing unit 12. Any of various approaches may be adopted for the extraction of information related to heartbeat by the extraction unit 13. The information related to heartbeat extracted by the extraction unit 13 may be supplied to the calculation unit 14.

The calculation unit 14 performs various arithmetic processing, computational processing, and/or the like on information related to heartbeat supplied from the extraction unit 13. Any of various approaches may be adopted for the various arithmetic processing, computational processing, and/or the like by the calculation unit 14. Various information resulting from the arithmetic processing, computational processing, and/or the like by the calculation unit 14 and/or the signal processing unit 10 may be supplied to the output unit 15 for example. For this reason, the calculation unit 14 and/or signal processing unit 10 may be connected to the output unit 15 for example. Various information resulting from the arithmetic processing, computational processing, and/or the like by the calculation unit 14 may also be supplied to another functional unit other than the output unit 15, such as a communication interface 50. Various information resulting from the arithmetic processing, computational processing, and/or the like by the calculation unit 14 may be supplied to, for example, a communication interface 50. For this reason, the calculation unit 14 and/or signal processing unit 10 may be connected to the communication interface 50. Various information resulting from the arithmetic processing, computational processing, and/or the like by the calculation unit 14 may also be supplied to another functional unit other than the communication interface 50.

The output unit 15 may output various information resulting from the arithmetic processing, computational processing, and/or the like by the calculation unit 14 to outside the signal processing unit 10, such as the communication interface 50 for example. For this reason, the output unit 15 may be connected to the communication interface 50 or the like. Information outputted from the output unit 15 may also be outputted to another functional unit other than the communication interface 50.

The output unit 15 may output a predetermined warning. In this case, the output unit 15 may output a predetermined warning if various information obtained as a result of arithmetic and/or computational processing by the calculation unit 14 meets a certain condition. The certain condition according to which a predetermined warning is outputted may be stored in the storage unit 16 for example. The output unit 15 may also output a predetermined response request. In this case, the output unit 15 may output a predetermined response request if various information obtained as a result of arithmetic and/or computational processing by the calculation unit 14 meets a certain condition. The certain condition according to which a predetermined response request is outputted may be stored in the storage unit 16 for example.

The storage unit 16 may function as a memory to store various information. The storage unit 16 may store, for example, a program to be executed in the signal processing unit 10 and the result of a process executed in the signal processing unit 10. The storage unit 16 may function as a working memory of the signal processing unit 10. For this reason, the storage unit 16 may be connected to the signal processing unit 10, the output unit 15, and/or the like in a wired and/or wireless manner. The storage unit 16 can be configured as a semiconductor memory, for example, but is not limited thereto, and can be any storage device. For example, the storage unit 16 may be a storage medium such as a memory card inserted into the electronic device 1 according to an embodiment. The storage unit 16 may also be an internal memory of a CPU or DSP to be used as the signal processing unit 10. The storage unit 16 may also be connected to the signal processing unit 10 as a separate unit.

The storage unit 16 may store information that serves as various criteria for determining whether the output unit 15 is to output various information. For example, the storage unit 16 may store information pertaining to a first criterion, which is at least one criterion related to heartbeat. The storage unit 16 may also store information pertaining to a second criterion, which is at least one criterion related to heartbeat. The information pertaining to the first criterion and the information pertaining to the second criterion will be described in further detail later.

The communication interface 50 is configured to include an interface that outputs information supplied from the signal processing unit 10 to, for example, an external device 60. The communication interface 50 may output information on at least one of the position, velocity, and angle of an object such as the subject of monitoring 200 as a controller area network (CAN) or other type of signal, for example, to the external device 60 or the like. For example, information on at least one of the position, velocity, and angle of an object such as the subject of monitoring 200 may be supplied to the external device 60 or the like via the communication interface 50. For this reason, the communication interface 50 may be connected to the external device 60 or the like.

The communication interface 50 may also supply information related to the heartbeat of the subject of monitoring 200 to the external device 60 for example. The communication interface 50 may also supply a predetermined warning, a predetermined response request, and/or the like outputted from the output unit 16. The communication interface 50 may also receive a response to the predetermined response request from the external device 60 for example.

As illustrated in FIG. 2, in an embodiment, the electronic device 1 may be connected to the external device 60 in a wired or wireless manner via the communication interface 50. In an embodiment, the external device 60 may be configured to include a computer of any kind, a control device of any kind, and/or the like. In an embodiment, the electronic device 1 may be configured to include the external device 60. The external device 60 may be configured in a variety of ways according to the manner in which information on heartbeat and/or heart sound detected by the electronic device 1 is to be used. Accordingly, a more detailed description of the external device 60 is omitted.

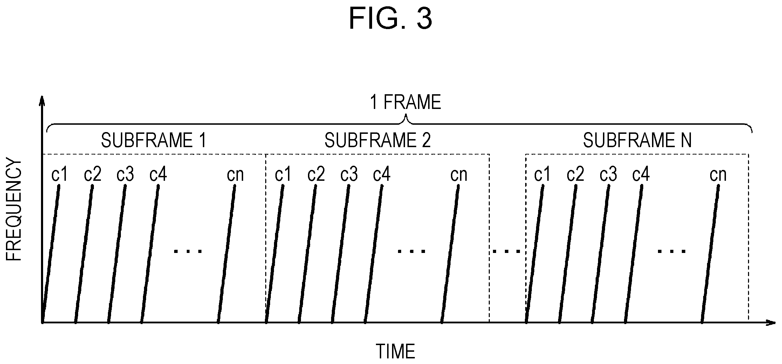

FIG. 3 is a diagram for describing an example of chirp signals generated by the signal generation processing unit 11 of the signal processing unit 10.

FIG. 3 illustrates the temporal structure of one frame in the case of using the fast-chirp modulation (FCM) scheme. FIG. 3 illustrates an example of a received signal of the FCM scheme. FCM is a scheme in which chirp signals illustrated as c1, c2, c3, c4, ..., cn in FIG. 3 are repeated at relatively short intervals (for example, equal to or greater than the round-trip time of electromagnetic waves between the radar and the target as calculated from the maximum ranging distance). In FCM, the transmission and reception processing is often divided into subframe units as illustrated in FIG. 3 for convenient signal processing of a received signal.

In FIG. 3, the horizontal axis represents elapsed time, and the vertical axis represents frequency. In the example illustrated in FIG. 3, the signal generation processing unit 11 generates linear chirp signals whose frequency varies periodically and linearly. In FIG. 3, the chirp signals are indicated as c1, c2, c3, c4, ..., cn. As illustrated in FIG. 3, in each of the chirp signals, the frequency increases linearly over time.

In the example illustrated in FIG. 3, several chirp signals such as c1, c2, c3, c4, ..., cn are included as a single subframe. That is, subframe 1, subframe 2, and so on illustrated in FIG. 3 are each configured to include several chirp signals such as c1, c2, c3, c4, ..., cn. In the example illustrated in FIG. 3, several subframes such as subframe 1, subframe 2, ..., subframe N are included as a single frame (1 frame). That is, the 1 frame illustrated in FIG. 3 is configured to include N subframes. The 1 frame illustrated in FIG. 3 may be frame 1, followed by frame 2, frame 3, and so on. These frames are each configured to include N subframes, in the same and/or similar manner as frame 1. A frame interval of given length may also be included between frames. The single frame illustrated in FIG. 3 may be around 30-50 milliseconds long, for example.

In the electronic device 1 according to an embodiment, the signal generation processing unit 11 may generate a transmission signal as any number of frames. In FIG. 3, some of the chirp signals are omitted from illustration. The relationship between the time and frequency of a transmission signal generated by the signal generation processing unit 11 in this way may be stored as various setting parameters in a storage unit or the like of the signal processing unit 10.

In this way, in an embodiment, the electronic device 1 may transmit a transmission signal formed from subframes including a plurality of chirp signals. In an embodiment, the electronic device 1 may transmit a transmission signal formed from frames including a given number of subframes.

The following describes the electronic device 1 as transmitting a transmission signal with a frame structure as illustrated in FIG. 3. However, the frame structure as illustrated in FIG. 3 is an example, and the chirp signals included in one subframes may be of any kind, for example. That is, in an embodiment, the signal generation processing unit 11 may generate subframes including any number of (for example, any plurality of) chirp signals. The subframe structure illustrated in FIG. 3 is also an example, and the subframes included in one frame may be of any kind, for example. That is, in an embodiment, the signal generation processing unit 11 may generate frames including any number of (for example, any plurality of) subframes. The signal generation processing unit 11 may generate signals of different frequency. The signal generation processing unit 11 may generate a plurality of discrete signals each having a different frequency f.

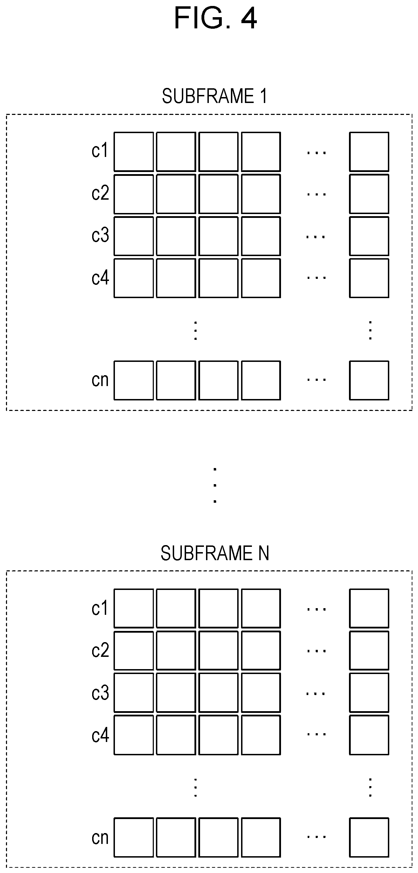

FIG. 4 illustrates another form of a portion of the subframes illustrated in FIG. 3. FIG. 4 is an illustration the result of the received signal processing unit 12 (FIG. 2) of the signal processing unit 10 performing two-dimensional fast Fourier transform (2D-FFT) processing on samples of a received signal obtained by receiving the transmission signal illustrated in FIG. 3.

As illustrated in FIG. 4, each of chirp signals c1, c2, c3, c4, ..., cn is stored in each of subframes such as subframe 1, ..., subframe N. In FIG. 4, each of the chirp signals c1, c2, c3, c4, ..., cn is formed from samples, which are indicated by the horizontally arrayed grid squares. The received signal illustrated in FIG. 4 is subjected to 2D-FFT, CFAR, integration signal processing on each subframe, and/or the like by the received signal processing unit 12 illustrated in FIG. 2.

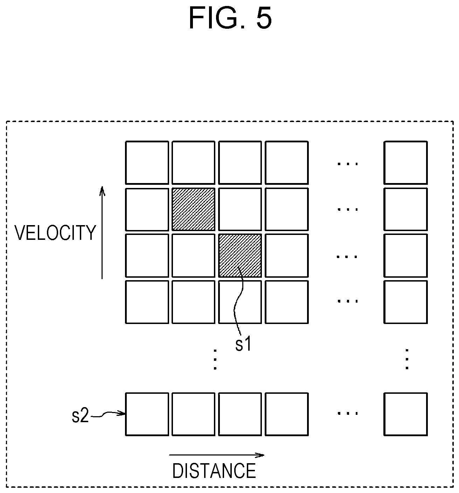

FIG. 5 illustrates an example of a point group in the range-Doppler (distance-velocity) plane calculated as a result of performing 2D-FFT, CFAR, and integration signal processing on each subframe in the received signal processing unit 12 illustrated in FIG. 2.

In FIG. 5, the horizontal direction represents range (distance), and the vertical direction represents velocity. The shaded grid square s1 illustrated in FIG. 5 illustrates a point group indicating a signal exceeding CFAR threshold processing. The non-shaded grid square s2 illustrated in FIG. 5 illustrates a bin (2D-FFT sample) with no point group, which did not exceed the CFAR threshold. Direction estimation is used to calculate the bearing, from the radar, of the calculated point group in the range-Doppler plane illustrated in FIG. 5, and the position and velocity in the two-dimensional plane are calculated as a point group indicating an object such as the subject of monitoring 200. Direction estimation may be calculated by a beamformer and/or a subspace method. Typical subspace method algorithms include MUltiple SIgnal Classification (MUSIC) and estimation of signal parameters via rotational invariant techniques (ESPRIT).

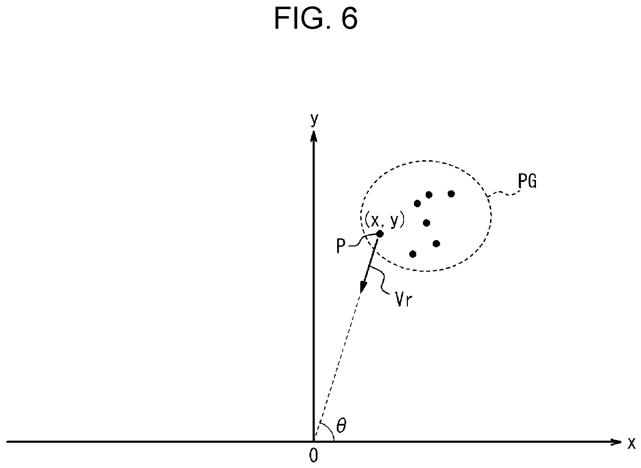

FIG. 6 illustrates an example of the result of the transformation of point group coordinates from the range-Doppler plane illustrated in FIG. 5 to the XY plane by the received signal processing unit 12 after performing direction estimation. The XY plane illustrated in FIG. 6 may be the same as the XY plane illustrated in FIG. 1. As illustrated in FIG. 6, the received signal processing unit 12 can plot a point group PG in the XY plane. The point group PG contains points P. Each of the points P has an angle θ and a radial velocity Vr in polar coordinates.

The received signal processing unit 12 detects an object present in the range where a transmission wave was transmitted, on the basis of at least one of the 2D-FFT or angle estimation results. The received signal processing unit 12 may perform object detection by performing, for example, clustering processing on the basis of respectively estimated information on distance, information on velocity, and angle information. Known algorithms used in clustering data include density-based spatial clustering of applications with noise (DBSCAN), for example. DBSCAN is an algorithm that performs density-based clustering. In the clustering processing, the average power of the points making up a detected object may be calculated, for example. The information on distance, information on velocity, angle information, and information on power pertaining to an object detected in the received signal processing unit 12 may be supplied to the external device 60 or the like via the communication interface 50, for example.

As above, the electronic device 1 may be provided with a transmitting antenna (transmitting antenna array 24), a receiving antenna (receiving antenna array 31), and the signal processing unit 10. The transmitting antenna array 24 transmits a transmission wave formed from a radio wave, for example. The receiving antenna array 31 receives a reflected wave resulting from the transmission wave being reflected. The signal processing unit 10 detects an object (an object such as the subject of monitoring 200, for example) that reflects the transmission wave, on the basis of the transmission signal transmitted as the transmission wave and the received signal received as the reflected wave.

By detecting change over time in the displacement of an object such as the subject of monitoring 200 in this way, the signal processing unit 10 can detect the velocity at which the object is displaced. If the surface of the object is vibrating slightly, as is the case for the chest area of the subject of monitoring 200 for example, the velocity at which the object vibrates can also be detected.

The following further describes direction estimation of an arriving wave (an arriving reflected wave) by an antenna array of the electronic device 1 according to an embodiment.

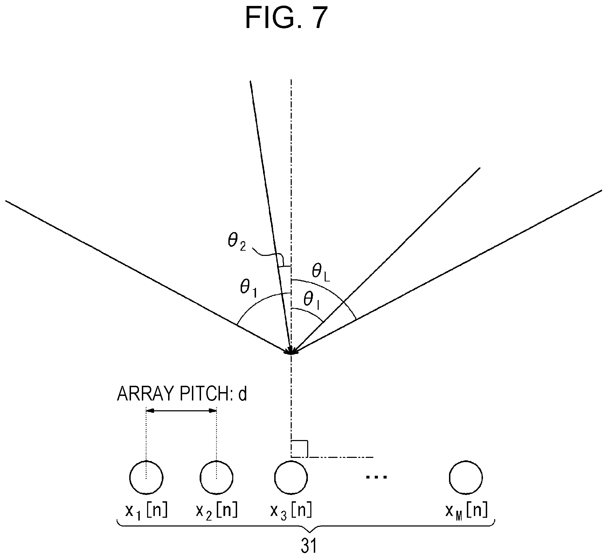

FIG. 7 is a diagram for describing the configuration of the receiving antenna array 31 and the principle of direction estimation of an arriving wave by the receiving antenna array 31 of the electronic device 1 according to an embodiment. FIG. 7 illustrates an example of the reception of radio waves by the receiving antenna array 31.

As illustrated in FIG. 7, the receiving antenna array 31 may be a linear arrangement of sensors such as receiving antennas. As illustrated in FIG. 7, in an embodiment, the receiving antenna array 31 may be configured to include a plurality of receiving antennas in a linear array. In FIG. 7, the plurality of antennas x₁, x₂, x₃, ..., x_{M} that make up the receiving antenna array 31 are illustrated by small circles. The receiving antenna array 31 may include any plurality of antennas. As illustrated in FIG. 7, the plurality of antennas that make up the receiving antenna array 31 are spaced apart from one another by an array pitch d. This type of sensor array, in which sensors (such as antennas, ultrasonic transducers, and microphones) corresponding to various physical waves are disposed in an array, is also referred to as a uniform linear array (ULA). As illustrated in FIG. 7, the physical waves (such as electromagnetic waves and sonic waves) arrive from various directions, such as θ₁ and θ₂, for example. Herein, θ₁ and θ₂ may refer to the angle of arrival described above. In this way, a sensor array like the receiving antenna array 31 can estimate the direction of arrival (angle of arrival) by utilizing the phase difference that occurs in measurement values between sensors according to the direction of arrival of a physical wave. This type of technique for estimating the direction of arrival of a wave is also referred to as angle-of-arrival estimation or direction-of-arrival (DoA) estimation.

In the electronic device 1 according to an embodiment, at least one of the transmitting antenna array 24 or the receiving antenna array 31 may have a plurality of antennas in a linear arrangement. This allows for appropriate narrowing of directivity in the transmission and reception of radio waves in a millimeter-wave radar, for example. When transmitting a transmission wave, the direction of the transmission beam is often controlled by a beamformer. On the other hand, when receiving a reflected wave, the direction of arrival of the reflected wave is more often estimated by subspace methods (such as MUSIC and ESPRIT described above) than by a beamformer. With beamformers and subspace methods, for electromagnetic waves arriving from various directions in the ULA as illustrated in FIG. 7, a phase difference occurs in measurement values between sensors depending on the direction of arrival. Accordingly, this phase difference can be utilized to estimate the direction of arrival of a reflected wave.

The following further describes angle estimation in two directions of an arriving wave by an antenna array of the electronic device 1 according to an embodiment.

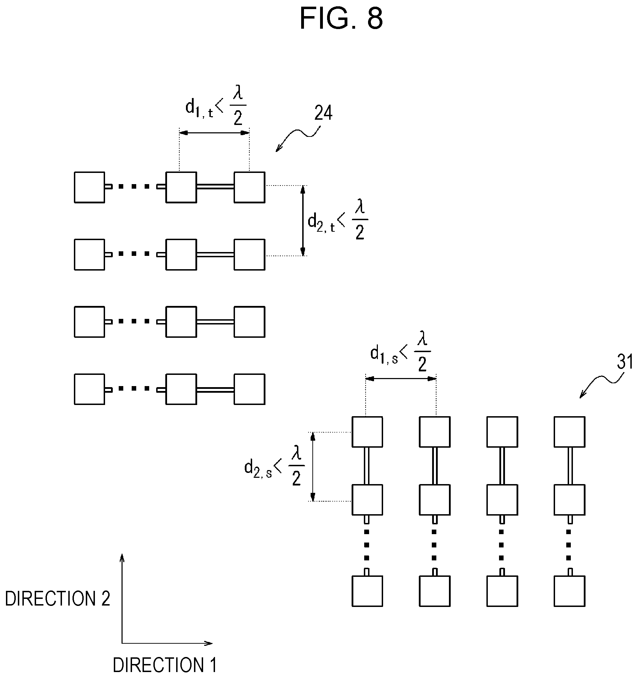

FIG. 8 illustrates an example arrangement of antennas for estimating the direction of arrival with respect to two orthogonal angles.

As illustrated in FIG. 8, in the electronic device 1 according to an embodiment, the transmitting antenna array 24 and/or receiving antenna array 31 may be configured to include an array of a plurality of patch antenna units.

In the transmitting antenna array 24 illustrated in FIG. 8, one patch antenna unit may be configured to include a plurality of elements electrically connected in the direction of direction 1 illustrated in the diagram. In each of the patch antenna units, the plurality of elements may be electrically connected by wiring such as striplines on a board, for example. In each of the patch antenna units, the plurality of elements may be spaced apart from one another by a pitch d_{1,t} shorter than half the wavelength λ of the transmission wave. In FIG. 8, each of the patch antenna units may have any number of two or more elements electrically connected.

As illustrated in FIG. 8, the transmitting antenna array 24 may include a plurality of patch antenna units arrayed in the direction of direction 2 illustrated in the diagram. The patch antenna units may be spaced apart from one another by a pitch d_{2,t} shorter than half the wavelength λ of the transmission wave. In an embodiment, the transmitting antenna array 24 may include any number of two or more patch antenna units.

As illustrated in FIG. 8, in an embodiment, the receiving antenna array 31 may be a variation of the arrangement of the plurality of elements in the transmitting antenna array 24. That is, in the receiving antenna array 31 illustrated in FIG. 8, one patch antenna unit may be configured to include a plurality of elements electrically connected in the direction of direction 2 illustrated in the diagram. In each of the patch antenna units, the plurality of elements may be electrically connected by wiring such as striplines on a board, for example. In each of the patch antenna units, the plurality of elements may be spaced apart from one another by a pitch d_{2,s} shorter than half the wavelength λ of the transmission wave. In FIG. 8, each of the patch antenna units may have any number of two or more elements electrically connected.

As illustrated in FIG. 8, the receiving antenna array 31 may include a plurality of patch antenna units arrayed in the direction of direction 1 illustrated in the diagram. The patch antenna units may be spaced apart from one another by a pitch d_{1,s} shorter than half the wavelength λ of the transmission wave. In an embodiment, the receiving antenna array 31 may include any number of two or more patch antenna units.

The elements included in the transmitting antenna array 24 and the receiving antenna array 31 may all be arranged in the same plane (on the surface layer of the same board, for example). The transmitting antenna array 24 and the receiving antenna array 31 may also be arranged in proximity to each other (monostatic). Direction 1 and direction 2 illustrated in FIG. 8 may be geometrically orthogonal.

With the transmitting antenna array 24 and the receiving antenna array 31 as illustrated in FIG. 8, the directivity of each of the transmitting antenna and the receiving antenna can be narrowed appropriately. By using the transmitting antenna array 24 as illustrated in FIG. 8 to control the direction in which to transmit each transmission wave at each timing for transmitting a transmission wave (transmission signal), a beamformer can be realized for the direction of direction 2 illustrated in FIG. 8. By using the receiving antenna array 31 as illustrated in FIG. 8, estimation of the direction of arrival of the reflected wave can be realized for the direction of direction 1 illustrated in FIG. 8. This enables estimation of the direction of arrival of a reflected wave with respect to two substantially orthogonal angles. Accordingly, a point group indicating an object such as the subject of monitoring 200 can be acquired three-dimensionally.

The following describes a technique for detecting the heartbeat of the subject of monitoring 200 by using the electronic device 1 according to an embodiment.

In an embodiment, the electronic device 1 can measure (estimate) the heartbeat of the subject of monitoring 200 on the basis of the result of transmitting a millimeter-wave radar or other transmission wave toward the subject of monitoring 200 and receiving a reflected wave that was reflected in the chest area of the subject of monitoring 200 where the heart resides. As described above, the subject of monitoring 200 may be a human being, and may also be an animal. In this case, for example, the vibrations at the position where the subject of monitoring 200 is detected to be present by the radar can be filtered by frequency to extract components assumed to be the envelope curve of the heartbeat. Once the components assumed to be the envelope curve of the heartbeat are extracted, the peak-to-peak intervals of the envelope curve can be obtained as the heartbeat interval to roughly calculate the heartbeat interval. The approximation that peaks of the heartbeat envelope roughly correspond to R peaks in an electrocardiogram can be used. Therefore, the "heartbeat interval" may also be referred to as the RR interval (or RRI), like the terms used for electrocardiograms and the like. In an embodiment, the electronic device 1 may also estimate the heartbeat interval of the subject of monitoring 200 from the result of performing the 2D-FFT, CFAR processing, and direction-of-arrival estimation described above and the like.

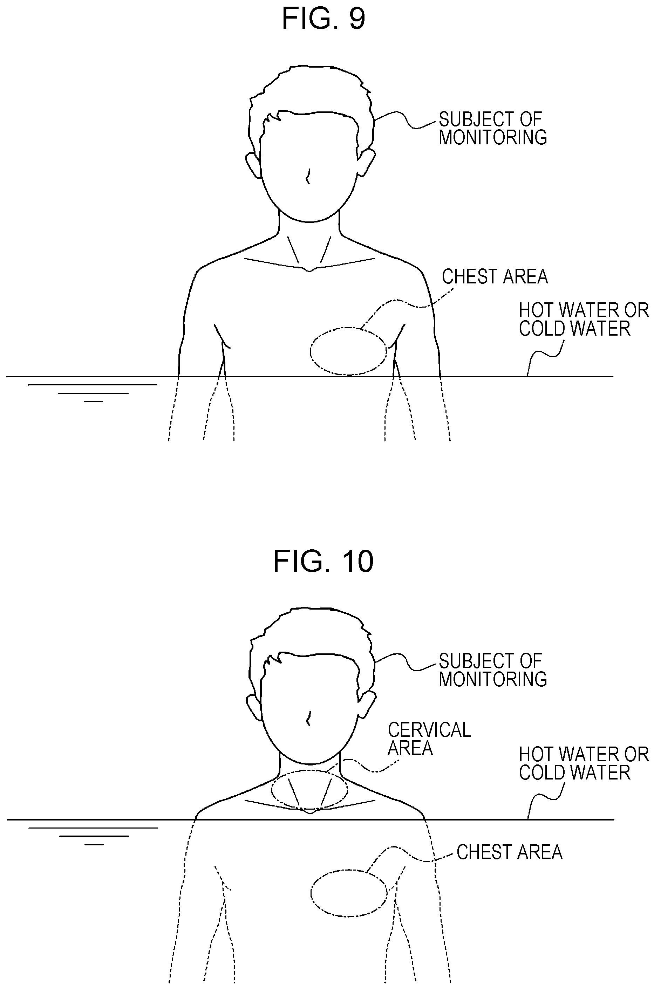

The following describes an environment in which the electronic device 1 according to an embodiment is used. FIGs. 9 and 10 are diagrams illustrating an example of a usage environment of the electronic device 1 according to an embodiment.

FIG. 9 illustrates a situation in which a portion of the body of a subject of monitoring, specifically a human being, is partially submerged in hot water or cold water, such as in a bathtub or a pool for example. FIG. 9 illustrates a situation in which the portion below the chest area of the subject of monitoring, specifically a human being, is submerged in hot water or cold water.

In an environment like the one illustrated in FIG. 9, the electronic device 1 according to an embodiment can detect vibrations in the chest area of the subject of monitoring by emitting a transmission wave at a body portion that includes the chest area of the subject of monitoring and receiving a reflected wave resulting from the transmission wave being reflected at the chest area of the subject of monitoring. In this way, in an environment like the one illustrated in FIG. 9, the electronic device 1 can generate information related to the heartbeat of the subject of monitoring on the basis of vibrations in the chest area of the subject of monitoring. That is, in an embodiment, the electronic device 1 can output information related to the heartbeat of the subject of monitoring even if a portion of the body (the portion below the chest area) of the subject of monitoring is submerged in hot water or cold water. In the present disclosure, "chest area" may refer to the portion of the human body or an animal between the neck and the abdomen. The chest area may include the surface portion and the internal portion of the body.

On the other hand, FIG. 10 illustrates a situation in which a portion of the body of a subject of monitoring, specifically a human being, is partially submerged in hot water or cold water, such as in a bathtub or a pool for example, deeper than in the illustration in FIG. 9. FIG. 10 illustrates a situation in which the subject of monitoring, specifically a human being, is submerged in hot water or cold water up to a portion above the chest area.

In an environment like the one illustrated in FIG. 10, the chest area of the human being who is the subject of monitoring is obscured beneath the water surface. In such an environment, the assumption is that even if the electronic device 1 emits a transmission wave at a body portion that includes the chest area of the subject of monitoring, a reflected wave resulting from the transmission wave being reflected at the chest area of the subject of monitoring cannot be received. In this case, vibrations in the chest area of the subject of monitoring cannot be detected. In this way, in an environment like the one illustrated in FIG. 10, the electronic device 1 may be unable to generate information related to the heartbeat of the subject of monitoring on the basis of vibrations in the chest area of the subject of monitoring. That is, in an embodiment, the electronic device 1 may be unable to output information related to the heartbeat of the subject of monitoring in a state where the part from which vibrations are to be detected is submerged in hot water or cold water.

However, the applicant has confirmed through demonstration experiments that even in environments like the one illustrated in FIG. 10, if the transmission wave is emitted so as to also include a cervical area of the subject of monitoring, a reflected wave resulting from the transmission wave being reflected in the cervical area of the subject of monitoring can be received. The applicant has confirmed that, in an embodiment, the electronic device 1 can receive such a reflected wave to thereby detect vibrations in the cervical area of the subject of monitoring. The "cervical area" may refer to the portion of a subject of monitoring that connects a head area and a chest area, and typically may be the portion of the neck. In this way, in an environment like the one illustrated in FIG. 10, the electronic device 1 can generate information related to the heartbeat of the subject of monitoring on the basis of vibrations in the chest area of the subject of monitoring and/or vibrations in the cervical area of the subject of monitoring. That is, in an embodiment, the electronic device 1 can output information related to the heartbeat of the subject of monitoring even if the subject of monitoring is submerged up to a portion of the body above the chest area in hot water or cold water.

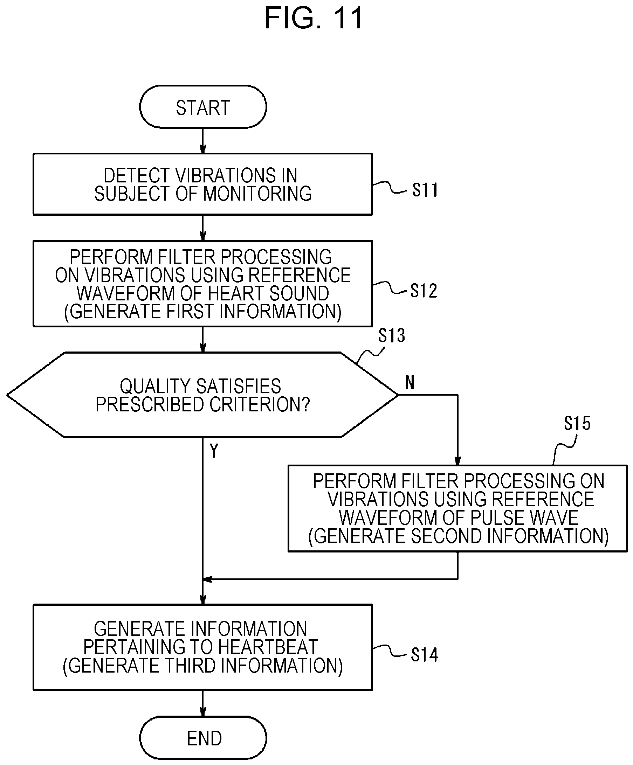

FIG. 11 is a flowchart for describing operations by the electronic device 1 according to an embodiment. The following refers to FIG. 11 to further describe operation by the electronic device 1 according to an embodiment.

The operations illustrated in FIG. 11 may be started when the electronic device 1 according to an embodiment is to output information related to the heartbeat of the subject of monitoring 200.

When the operations illustrated in FIG. 11 start, the signal processing unit 10 detects vibrations in the subject of monitoring 200 (step S11). In step S11, the signal processing unit 10 may detect vibrations in the subject of monitoring 200 on the basis of a transmission signal that the electronic device 1 transmits as a transmission wave and a received signal received as a reflected wave resulting from the transmission wave being reflected by the subject of monitoring. In step S11, the electronic device 1 may transmit a transmission wave having an emission angle that includes the chest area and the cervical area of the subject of monitoring 200. In this way, the signal processing unit 10 may detect vibrations in the chest area and/or the cervical area of the subject of monitoring 200.

In step S11, the signal processing unit 10 may detect vibrations in the subject of monitoring 200 on the basis of a transmission signal transmitted by a single sensor (for example, a radar sensor) and a received signal received by the single sensor. Through step S11, the signal processing unit 10 may collectively detect vibrations in various parts of the body of the subject of monitoring 200, including the chest area and/or the cervical area of the subject of monitoring 200.

The signal processing unit 10 generates (or extracts) information related to the heart sound of the subject of monitoring 200 on the basis of vibrations in a first part (for example, the chest area) of the subject of monitoring 200 (step S12). The information related to the heart sound of the subject of monitoring 200 that is generated (or extracted) in step S12 is hereinafter also referred to as "first information". In step S12, the signal processing unit 10 may generate the first information by using a heart sound reference waveform to perform filter processing on the vibrations detected in the first part (for example, the chest area). For example, the signal processing unit 10 may generate the first information by comparing vibrations (a heart sound waveform) in the first part (for example, the chest area) and a heart sound waveform acquired in advance that serves as a reference. The signal processing unit 10 may also generate the first information on the basis of vibrations in the first part (for example, the chest area) by performing prescribed processing such as pattern matching (processing for determining whether the difference from a pattern acquired in advance is within a prescribed range). Otherwise, the signal processing unit 10 may use any of various approaches to generate the first information on the basis of vibrations in the first part (for example, the chest area).

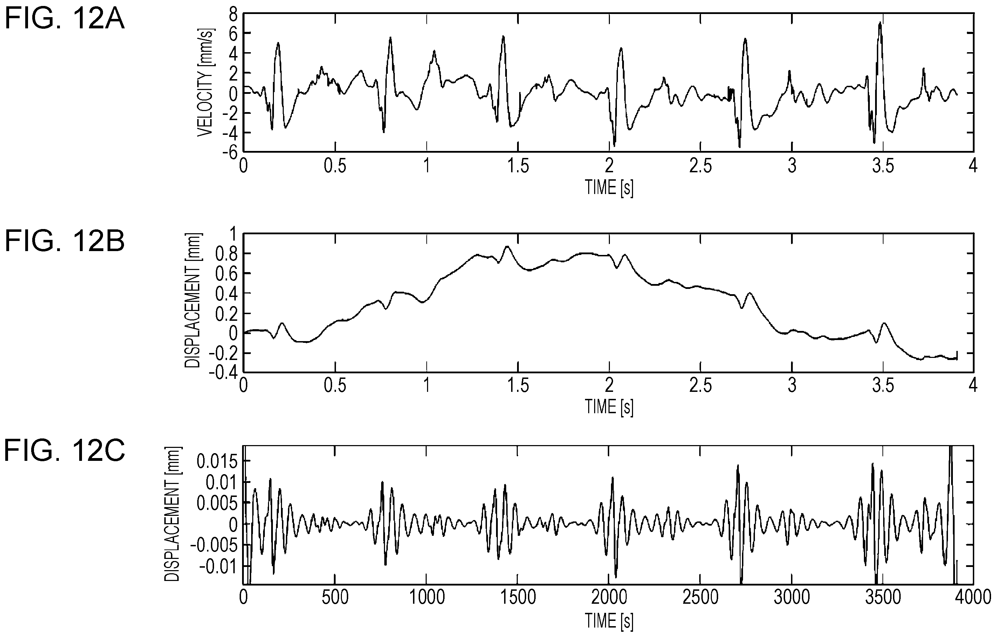

FIGs. 12A to 12C are diagrams illustrating an example of vibrations in the first part (chest area) of the subject of monitoring 200 generated by the signal processing unit 10.

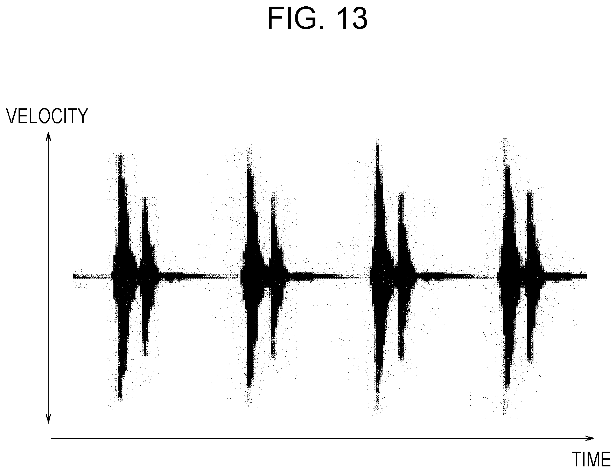

FIG. 12A is a graph illustrating an example of change over time in the velocity of vibrations in the first part (chest area) of the subject of monitoring 200. In an embodiment, the electronic device 1 can directly detect change over time in the velocity of vibrations like that illustrated in FIG. 12A. FIG. 13 is a diagram illustrating an enlarged view of a portion of the graph illustrated in FIG. 12A. As illustrated in FIG. 13, change over time in the velocity of vibrations in the first part (chest area) of the subject of monitoring 200 constitutes a heart sound waveform that includes definite peaks of a first sound and a second sound.

FIG. 12B is a graph illustrating an example of change over time in the displacement of vibrations in the first part (chest area) of the subject of monitoring 200. The graph illustrated in FIG. 12B can be acquired on the basis of FIG. 12A, for example. The graph illustrated in FIG. 12C illustrates an example of change over time in the displacement of vibrations in the first part (chest area) of the subject of monitoring 200 (for example, FIG. 12B) over a relatively long period.

In step S12, the signal processing unit 10 may extract a heart sound waveform (generate the first information) that can be determined to be relatively good from at least one of the graphs like those illustrated in FIGs. 12A to 12C described above.

The signal processing unit 10 determines whether the generated first information satisfies a prescribed quality (step S13). In step S13, the signal processing unit 10 may compare the waveform expressed by the first information to a preset heart sound waveform, and thereby determine that the first information "satisfies a prescribed quality" if the difference between the two waveforms is within a prescribed range. That is, in step S13, the signal processing unit 10 may determine whether the waveform expressed by the first information is suitable as a heart sound waveform. In step S13, the signal processing unit 10 may determine whether the first information satisfies the prescribed quality by using any of various approaches. The case where the first information satisfies the prescribed quality may be the case where heart sound can be acquired appropriately on the basis of vibrations in the chest area of the test subject, like the case illustrated in FIG. 9 for example.

In step S13, if the first information satisfies the prescribed quality (step S13, Yes), (the output unit 16 of) the signal processing unit 10 may generate information related to the heartbeat of the subject of monitoring 200 (step S14). The information related to the heartbeat of the subject of monitoring 200 that is generated in step S14 is hereinafter also referred to as "third information". The third information may be the heart rate, for example. In the case of advancing to step S14 from step S13, the signal processing unit 10 may generate the third information on the basis of the first information.

On the other hand, in step S13, if the first information does not satisfy the prescribed quality (step S13, No), the signal processing unit 10 performs the processing in step S15. The case where the first information does not satisfy the prescribed quality may be the case where heart sound cannot be acquired appropriately on the basis of vibrations in the chest area of the test subject, like the case illustrated in FIG. 10 for example. In step S15, the signal processing unit 10 generates (or extracts) information related to the pulse wave of the subject of monitoring 200 on the basis of vibrations in a second part (for example, the cervical area) of the subject of monitoring 200. The information related to the pulse wave of the subject of monitoring 200 that is generated (or extracted) in step S15 is hereinafter also referred to as "second information". In step S15, the signal processing unit 10 may generate the second information by using a pulse wave reference waveform to perform filter processing on the vibrations detected in the second part (for example, the cervical area). For example, the signal processing unit 10 may generate the second information by comparing vibrations (a pulse wave waveform) detected in the second part (for example, the cervical area) and a pulse wave waveform acquired in advance that serves as a reference. The signal processing unit 10 may also generate the second information on the basis of vibrations in the second part (for example, the cervical area) by performing prescribed processing such as pattern matching (processing for determining whether the difference from a pattern acquired in advance is within a prescribed range). Otherwise, the signal processing unit 10 may use any of various approaches to generate the second information on the basis of vibrations in the second part (for example, the cervical area).

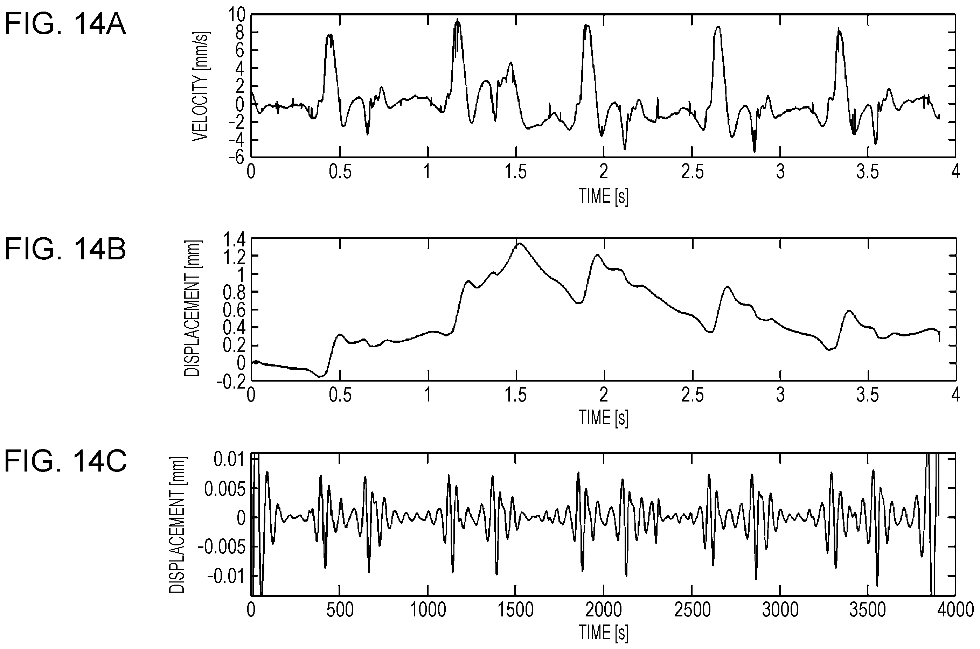

FIGs. 14A to 14C are diagrams illustrating an example of vibrations in the second part (cervical area) of the subject of monitoring 200 generated by the signal processing unit 10.

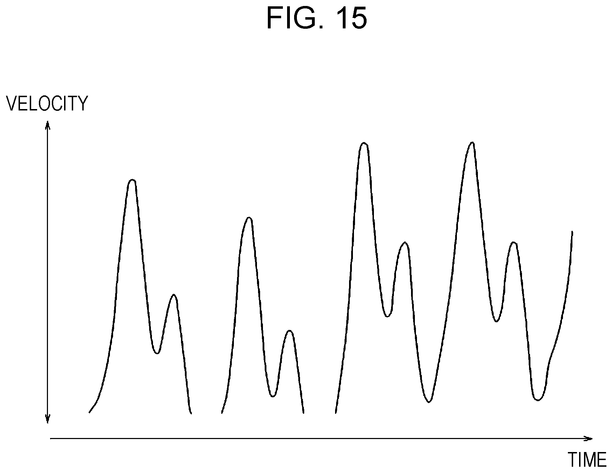

FIG. 14A is a graph illustrating an example of change over time in the velocity of vibrations in the second part (cervical area) of the subject of monitoring 200. In an embodiment, the electronic device 1 can directly detect change over time in the velocity of vibrations like that illustrated in FIG. 14A. FIG. 15 is a diagram illustrating an enlarged view of a portion of the graph illustrated in FIG. 14A. As illustrated in FIG. 15, change over time in the velocity of vibrations in the second part (cervical area) of the subject of monitoring 200 constitutes a pulse wave waveform that does not include definite peaks such as of a first sound and a second sound.

FIG. 14B is a graph illustrating an example of change over time in the displacement of vibrations in the second part (cervical area) of the subject of monitoring 200. The graph illustrated in FIG. 14B can be acquired on the basis of FIG. 14A, for example. The graph illustrated in FIG. 14C illustrates an example of change over time in the displacement of vibrations in the second part (cervical area) of the subject of monitoring 200 (for example, FIG. 14B) over a relatively long period.

In step S15, the signal processing unit 10 may extract a pulse wave waveform (generate the second information) that can be determined to be relatively good from at least one of the graphs like those illustrated in FIGs. 14A to 14C described above.

In step S15, (the output unit 16 of) the signal processing unit 10 may generate information (third information) related to the heartbeat of the subject of monitoring 200 (step S14). In the case of advancing to step S14 from step S15, the signal processing unit 10 may generate the third information on the basis of the first information and/or the second information. For example, if no first information at all can be obtained, or if the quality of the first information is poor to a certain extent, the signal processing unit 10 may generate the third information on the sole basis of the second information. On the other hand, if the quality of the first information cannot be said to be poor to a certain extent, the signal processing unit 10 may generate the third information on the basis of both the first information and the second information. The signal processing unit 10 may also generate the third information on the sole basis of the second information, even if the quality of the first information cannot be said to be poor to a certain extent.

As described above, in the electronic device 1 according to an embodiment, the signal processing unit 10 generates (or extracts) first information related to the heart sound of the subject of monitoring 200 on the basis of vibrations in a first part of the subject of monitoring 200. If the first information does not satisfy a prescribed criterion, the signal processing unit 10 generates (or extracts) second information related to the pulse wave of the subject of monitoring 200 on the basis of vibrations in a second part, which is different from the first part, of the subject of monitoring 200. The signal processing unit 10 may also generate third information related to the heartbeat of the subject of monitoring 200 on the basis of the first information and/or the second information.

In an embodiment, the first part may be a prescribed part of the body of the subject of monitoring 200. The second part may be a part of the subject of monitoring 200 located above the first part. More specifically, in an embodiment, the first part may be a chest area of the subject of monitoring 200. The second part may be a cervical area of the subject of monitoring 200.

According to an embodiment, the electronic device 1 is expected to be capable of appropriately acquiring information related to the heartbeat of a subject of monitoring, even in a situation like the one illustrated in FIG. 9 and even in a situation like the one illustrated in FIG. 10, for example. Consequently, according to an embodiment, the electronic device 1 has an increased likelihood of detecting biological information such as the heartbeat of a subject of monitoring by transmitting and receiving radio waves. The ability to increase the likelihood of detecting biological information such as the heartbeat of a subject of monitoring by transmitting and receiving radio waves could be useful in a wide variety of fields.

### (Other embodiments)

The following describes other embodiments.

According to an embodiment, if a subject of monitoring is determined to be at risk as a result of measuring the heartbeat of the subject of monitoring, the electronic device 1 may output a warning or the like to that effect to the subject of monitoring or to a third party. For the purpose of such processing, the storage unit 16 may store information pertaining to a first criterion, which is at least one criterion related to heartbeat. The output unit 15 of the signal processing unit 10 may output a prescribed warning on the basis of the heartbeat of the subject of monitoring 200 indicated by the third information being equal to or greater than the first criterion. In this case, the first criterion may be set according to the subject of monitoring 200. That is, the first criterion may be set differently for each subject of monitoring.

According to an embodiment, if a subject of monitoring is determined to be at risk as a result of measuring the heartbeat of the subject of monitoring, the electronic device 1 may output a request (response request) or the like asking the subject of monitoring or a third party to provide some kind of response. For the purpose of such processing, the storage unit 16 may store information pertaining to a second criterion, which is at least one criterion related to heartbeat. The output unit 15 of the signal processing unit 10 may output a prescribed response request on the basis of the heartbeat of the subject of monitoring indicated by the third information being equal to or greater than the second criterion. In this case, the second criterion may be set according to the subject of monitoring 200. That is, the second criterion may be set differently for each subject of monitoring.

The signal processing unit 10 may also execute prescribed processing, such as automatically requesting ambulance transportation for example, if a response to the prescribed response request described above is not received within a prescribed time (such as 10 minutes).

In the electronic device 1 illustrated in FIG. 2, the signal processing unit 10 is described as being provided with functional units such as the extraction unit 13, the calculation unit 14, the output unit 15, and the storage unit 16. However, in an embodiment, the processing performed by the extraction unit 13, the calculation unit 14, and/or the output unit 15 may also be performed by an external computer, processor, or the like. The storage unit 16,

As described above, in an embodiment, the electronic device 1 uses, for example, a millimeter-wave sensor provided with a plurality of transmitting antennas and a plurality of receiving antennas to detect weak vibrations such as a heartbeat. In an embodiment, when the subject of monitoring is not detected, the electronic device 1 detects the body motion of the subject of monitoring while varying the transmission phase of the antennas to create a beamforming pattern of the transmitting antennas. On the other hand, in an embodiment, once the body motion of the subject of monitoring is detected, the electronic device 1 carries out beamforming in the direction of the body motion to detect the heartbeat. In this way, according to an embodiment, the electronic device 1 can improve the signal quality by automatically detecting the direction of a human body. Consequently, according to an embodiment, the electronic device 1 can improve the heartbeat detection precision and/or detection range. Thus, according to an embodiment, the electronic device 1 can detect the heartbeat of a human being with high precision.

The present disclosure has been described on the basis of the drawings and examples, but note that a person skilled in the art could easily make various variations or revisions on the basis of the present disclosure. Consequently, it should be understood that these variations or revisions are included in the scope of the present disclosure. For example, the functions and the like included in each functional unit may be rearranged in logically non-contradictory ways. Multiple functional units or the like may be combined into one, or a functional unit may be divided. Each embodiment according to the present disclosure described above is not limited to being carried out exactly according to each embodiment as described, and may be carried out by combining features or omitting some features, as appropriate. In other words, the content of the present disclosure enables a person skilled in the art to make various variations and revisions on the basis of the present disclosure. Therefore, these variations and revisions are included in the scope of the present disclosure. For example, in each embodiment, each functional unit, means, step, and the like can be added to another embodiment or replaced by each functional unit, means, step, and the like of another embodiment in logically non-contradictory ways. In each embodiment, multiple functional units, means, steps, and the like can be combined into one, or each functional unit, means, step, and the like can be divided. Each embodiment according to the present disclosure described above is not limited to being carried out exactly according to each embodiment as described, and can be carried out by combining features or omitting some features, as appropriate.

The embodiments described above are not limited solely to embodiments of the electronic device 1. For example, the embodiments described above may also be carried out as a method of controlling a device like the electronic device 1. Furthermore, the embodiments described above may also be carried out as a program to be executed by a device like the electronic device 1, for example, or as a storage medium or recording medium in which the program is recorded.

In the embodiments described above, the electronic device 1 is described as including components such as the transmitting antenna array 24 and the receiving antenna array 31 that form what is called a radar sensor. However, in an embodiment, the electronic device may be carried out as a configuration like the signal processing unit 10, for example. In this case, the signal processing unit 10 may be carried out as a unit having functions for processing signals handled by the transmitting antenna array 24, the receiving antenna array 31, and the like.

### REFERENCE SIGNS

- 1: electronic device
- 10: signal processing unit
- 11: signal generation processing unit
- 12: received signal processing unit
- 13: extraction unit
- 14: calculation unit
- 15: output unit
- 16: storage unit
- 21: transmission DAC
- 22: transmission circuit
- 23: millimeter-wave transmission circuit
- 24: transmitting antenna array
- 31: receiving antenna array
- 32: mixer
- 33: reception circuit
- 34: reception ADC
- 50: communication interface
- 60: external device
- 70: audiovisual device

## Claims

1. An electronic device comprising a signal processing unit configured to detect vibrations in a subject of monitoring on the basis of a transmission signal transmitted as a transmission wave and a received signal received as a reflected wave resulting from the transmission wave being reflected by the subject of monitoring,
the signal processing unit being configured to
generate first information related to a heart sound of the subject of monitoring on the basis of vibrations in a first part of the subject of monitoring, and
if the first information does not satisfy a prescribed criterion, generate second information related to a pulse wave of the subject of monitoring on the basis of vibrations in a second part, which is different from the first part, of the subject of monitoring.

2. The electronic device according to claim 1, wherein the signal processing unit is configured to generate third information related to a heartbeat of the subject of monitoring on the basis of the first information and/or the second information.

3. The electronic device according to claim 1, wherein the signal processing unit is configured to detect vibrations in the subject of monitoring on the basis of the transmission signal transmitted by a single sensor and the received signal received by the single sensor.

4. The electronic device according to claim 1, wherein
the first information is generated on the basis of vibrations in a prescribed part of the body of the subject of monitoring as the first part, and
the second information is generated on the basis of vibrations in a part of the body of the subject of monitoring located above the first part as the second part.

5. The electronic device according to claim 1, wherein
the first information is generated on the basis of vibrations in a chest area of the subject of monitoring as the first part, and
the second information is generated on the basis of vibrations in a cervical area of the subject of monitoring as the second part.

6. The electronic device according to claim 2, comprising:
a storage unit configured to store information pertaining to a first criterion, which is at least one criterion related to heartbeat; and
an output unit configured to output a prescribed warning on the basis of the heartbeat of the subject of monitoring indicated by the third information being equal to or greater than the first criterion.

7. The electronic device according to claim 6, wherein the first criterion is set according to the subject of monitoring.

8. The electronic device according to claim 2, comprising:
a storage unit configured to store information pertaining to a second criterion, which is at least one criterion related to heartbeat; and
an output unit configured to output a prescribed response request on the basis of the heartbeat of the subject of monitoring indicated by the third information being equal to or greater than the second criterion.

9. The electronic device according to claim 8, wherein the second criterion is set according to the subject of monitoring.

10. The electronic device according to claim 8, wherein the signal processing unit is configured to execute prescribed processing if a response to the prescribed response request is not received within a prescribed time.

11. A method of controlling an electronic device provided with a signal processing unit configured to detect vibrations in a subject of monitoring on the basis of a transmission signal transmitted as a transmission wave and a received signal received as a reflected wave resulting from the transmission wave being reflected by the subject of monitoring, the method comprising:
generating first information related to a heart sound of the subject of monitoring on the basis of vibrations in a first part of the subject of monitoring; and
generating, if the first information does not satisfy a prescribed criterion, second information related to a pulse wave of the subject of monitoring on the basis of vibrations in a second part, which is different from the first part, of the subject of monitoring.

12. A program causing an electronic device to execute processing, the electronic device being provided with a signal processing unit configured to detect vibrations in a subject of monitoring on the basis of a transmission signal transmitted as a transmission wave and a received signal received as a reflected wave resulting from the transmission wave being reflected by the subject of monitoring, the processing comprising:
generating first information related to a heart sound of the subject of monitoring on the basis of vibrations in a first part of the subject of monitoring; and
generating, if the first information does not satisfy a prescribed criterion, second information related to a pulse wave of the subject of monitoring on the basis of vibrations in a second part, which is different from the first part, of the subject of monitoring.
